# EUROPÄISCHE PATENTANMELDUNG

(11) **EP 4 497 451 A1**
(43) Veröffentlichungstag der Anmeldung: **29.01.2025**
(21) Anmeldenummer: 23187577.4
(22) Anmeldetag: 25.07.2023
(51) Int. Cl.: A61L 27/44, A61L 27/50, B32B 15/00, B32B 17/02

(54) **MEHRSCHICHTMATERIAL, VERFAHREN ZUR HERSTELLUNG DES MEHRSCHICHTMATERIALS UND ANWENDUNG DES MEHRSCHICHTMATERIALS SOWIE MEDIZINISCHES KARDIOVASKULÄRES IMPLANTAT AUFWEISEND DAS MEHRSCHICHTMATERIAL UND VERFAHREN ZUR HERSTELLUNG DES MEDIZINISCHEN KARDIOVASKULÄREN IMPLANTATS**

(71) Anmelder: Universität Rostock, 18055 Rostock (DE)
(72) Erfinder: Illner, Sabine, 18059 Rostock (DE); Götz, Andreas, 18109 Rostock (DE); Grabow, Niels, 18055 Rostock (DE); Strotmeier, Manfred, 18239 Satow OT Hanstorf (DE)
(74) Vertreter: Grünbaum, Annekathrin

(57) **Zusammenfassung**

Die Erfindung betrifft ein Mehrschichtmaterial, ein Verfahren zur Herstellung des Mehrschichtmaterials und eine Anwendung des Mehrschichtmaterials sowie ein medizinisches kardiovaskuläres Implantat aufweisend das Mehrschichtmaterial und ein Verfahren zur Herstellung des medizinischen kardiovaskulären Implantats.

Es ist daher Aufgabe der Erfindung die Nachteile des Standes der Technik zu beseitigen und ein Mehrschichtmaterial, ein Verfahren zur Herstellung des Mehrschichtmaterials und eine Anwendung des Mehrschichtmaterials bereitzustellen. Es ist ferner Aufgabe der Erfindung ein medizinisches kardiovaskuläres Implantat aufweisend das Mehrschichtmaterial und ein Verfahren zur Herstellung des medizinischen kardiovaskulären Implantats bereitzustellen, welches zuverlässig, langzeitstabil und kostengünstig ist.

Die Lösung dieser Aufgabe erfolgt durch die in den Ansprüchen aufgeführten Merkmale.

## Beschreibung

Die Erfindung betrifft ein Mehrschichtmaterial, ein Verfahren zur Herstellung des Mehrschichtmaterials und eine Anwendung des Mehrschichtmaterials sowie ein medizinisches kardiovaskuläres Implantat aufweisend das Mehrschichtmaterial und ein Verfahren zur Herstellung des medizinischen kardiovaskulären Implantats.

Das Herz kann an verschiedenen Klappenerkrankungen oder Fehlbildungen leiden, die zu erheblichen Fehlfunktionen des Herzens führen und letztendlich den Ersatz der natürlichen Herzklappe durch eine künstliche Klappe erfordern. Menschliche Herzklappen, zu denen die Aorten-, Pulmonal-, Mitral- und Trikuspidalklappe gehören, funktionieren im Wesentlichen als Einwegventile, die synchron mit dem pumpenden Herzen arbeiten. Die Klappen ermöglichen den Blutfluss stromabwärts, blockieren jedoch den Blutfluss stromaufwärts.

Erkrankte Herzklappen weisen Beeinträchtigungen wie eine Verengung der Klappe oder ein Aufstoßen auf, wodurch die Fähigkeit der Klappen, den Blutfluss zu kontrollieren, beeinträchtigt wird. Solche Beeinträchtigungen verringern die Blutpumpleistung des Herzens und können eine schwächende und lebensbedrohliche Erkrankung sein. Beispielsweise kann eine Klappeninsuffizienz zu Erkrankungen wie einer Herzhypertrophie und einer Erweiterung der Herzkammer führen. Daher wurden umfangreiche Anstrengungen unternommen, um Methoden und Geräte zur Reparatur oder zum Ersatz beeinträchtigter Herzklappen zu entwickeln.

Es gibt Prothesen zur Korrektur von Problemen im Zusammenhang mit Herzklappenstörungen. So können zum Beispiel mechanische und gewebebasierte Herzklappenprothesen eingesetzt werden, um beeinträchtigte, körpereigene Herzklappen zu ersetzen.

Bei mechanisch beanspruchten Implantaten im Blutkontakt, z.B. Herzklappenprothesen, lässt sich mit xenogenen oder alloplastischen (polymeren) Segelstrukturen meist nicht die gewünschte Langzeitstabilität erzielen.

Für den biologischen Herzklappenersatz wird üblicherweise xenogenes Material (meist porcines oder bovines Perikard) verwendet. Diese Prothesen sind kostenintensiv in der Herstellung und haben eine Lebensdauer meist unter 10 Jahren.

Für den mechanischen Herzklappenersatz werden artifizielle Materialien verwendet, die eine längere Lebensdauer aufweisen, aber neben einer nachteiligen Geräuschentwicklung beim Zuschlagen eine lebenslange Medikation erfordern.

Beim Implantationsvorgang selber spielen neben den offenchirurgischen Operationstechniken minimalinvasive Verfahren, wie beispielsweise die Transkatheter-Aortenklappenimplantation (TAVI), eine zunehmende Rolle.

Im Forschungs- und Entwicklungsbereich werden derzeit verschiedene Polymerverarbeitungs- und Beschichtungstechnologien zur Erzeugung artifizieller Segelmaterialien evaluiert. Beschichtungstechnologien umfassen u.a. Tauchbeschichtung, Sprühbeschichtung, Formpressen und Elektrospinning. Das Anforderungsprofil, welches umfangreiche Muss-Kriterien, wie gleichbleibende physikochemische Materialqualität, mechanische Dauerperformance und Crimpfähigkeit, sowie biologische Inertheit umfasst, wird nach gängigem Wissensstand bisher von keinem Polymermaterial allumfassend oder in ausreichendem Maße erfüllt. Dabei ist es unerheblich, ob die artifiziellen Segel strukturiert, faserförmig, porös oder kompakt, in Schichten oder Verbünden aufgebaut sind, denn insbesondere die Dauerperformance unter hohen Druckgradienten ist begrenzt.

Forschungsaktivitäten auf dem Gebiet der artifiziellen Ersatzmaterialien, insbesondere von thermoplastischen Polyurethanen (TPU) und deren Copolymeren sind bekannt, wobei u.a. die Hämokompatibilität, Fügung, langzeitstabile Verankerung in einem Stentgerüst, Langzeitstabilität, Dauerfestigkeit unter physiologischen Bedingungen, mechanische Performance, zelluläre Besiedelung, Degradationsprozesse, Kalzifikation sowie Delaminationseigenschaften polymerer Folien- oder Vliesklappenstrukturen erforscht werden.

In jüngsten Arbeiten werden darüber hinaus bereits regenerative Transkatheter-Aortenklappenprothesen bzw. mitwachsende Herzklappen aus biodegradierbaren Polymeren (PCL, PLLA) erforscht.

WO2021251974A1 offenbart synthetische Stoffmaterialien, die in verschiedenen medizinischen Geräten verwendet werden können.

US2022054702A1 offenbart ein chirurgisches Implantat auf Faserbasis, das gegen Ausfransen stabilisiert ist, und ein thermisch gekräuseltes Flachstrickgewebe aus einem biokompatiblen, optional biologisch abbaubaren, Polymermaterial umfasst.

US20220133950A1 offenbart ein biokompatibles Textil, bestehend aus mindestens einer elektrogesponnenen Faser, umfassend mindestens ein Polymer, wobei das biokompatible Textil einem Patienten transplantiert werden kann, um beschädigtes Gewebe zu ersetzen.

WO0197741A2 offenbart eine anatomisch geformte Atrioventrikularklappe bestehend aus einer biologisch kompatiblen synthetischen Membran oder einer biologischen Membran autologen, homologen oder heterologen Ursprungs.

WO2021173937A2 offenbart mehrere Ausführungsformen eines Blättchenmaterials, das in aktuellen und zukünftigen Herzklappenprothesen verwendet werden kann, einschließlich chirurgisch implantierter mechanischer Herzklappen und Transkatheter-Herzklappen. Eine solche Herzklappenprothese kann eine Stützstruktur umfassen und eine Klappenanordnung, die innerhalb der Stützstruktur angeordnet ist, wobei die Klappenanordnung mehrere Blättchen umfasst, wobei jedes Blättchen aus einem Verbundmaterial gebildet ist, wobei das Verbundmaterial ein Metallsubstrat mit mehreren Öffnungen und ein auf das Metallsubstrat aufgetragenes Polymer umfasst.

Trotz einer Fülle an Entwicklungsaktivitäten ist bis zum heutigen Zeitpunkt keine artifizielle Prothese bekannt, die eine vollumfänglich zufriedenstellende Performance hinsichtlich der oben genannten Gesichtspunkte zeigt.

Zahlreiche experimentelle Untersuchungen haben gezeigt, dass polymere Materialien sowohl als kompakte Folie, als hochporöses Faservliesmaterial, sowie als Kombinationsvariante (Hybrid, Komposit, Schichtverbund) in Form einer tricuspiden Herzklappenprothese unzureichende mechanische Belastbarkeit aufweisen. Die Nutzung polymerer Verstärkungsstrukturen zur lokalen Stabilisierung der Faservliesklappensegel sind Gegenstand aktueller Forschung. Aus bisherigen Ergebnissen zeichnet sich jedoch ab, dass in näherer Zukunft hiermit keine optimale Lösung erzielbar ist.

### Darstellung der Erfindung

Es ist Aufgabe der Erfindung die Nachteile des Standes der Technik zu beseitigen und ein Mehrschichtmaterial, ein Verfahren zur Herstellung des Mehrschichtmaterials und eine Anwendung des Mehrschichtmaterials bereitzustellen. Es ist ferner Aufgabe der Erfindung ein medizinisches kardiovaskuläres Implantat aufweisend das Mehrschichtmaterial und ein Verfahren zur Herstellung des medizinischen kardiovaskulären Implantats bereitzustellen, welches zuverlässig, langzeitstabil und kostengünstig ist.

Die Lösung dieser Aufgabe erfolgt durch die in den Ansprüchen aufgeführten Merkmale.

Die Lösung der Aufgabe erfolgt durch ein Mehrschichtmaterial, wobei das Mehrschichtmaterial zumindest zwei äußere Schichten und zumindest eine innere Schicht aufweist. Die erste äußere Schicht umfasst eine erste Polymerbeschichtung. Die zweite äußere Schicht umfasst eine zweite Polymerbeschichtung. Die innere Schicht umfasst ein flexibel ausgestaltetes Metallgebilde, welches metallisches Glas aufweist. Die Schichten sind mittels unlösbarer Verbindungen miteinander fixiert, wobei die innere Schicht zwischen der ersten äußeren Schicht und der zweiten äußeren Schicht angeordnet ist.

Gemäß verschiedener Ausführungsformen weist das Mehrschichtmaterial zumindest eine vordefinierte fixierte Falte auf.

Gemäß verschiedener Ausführungsformen sind die Polymerbeschichtungen der äußeren Schichten und/oder die äußeren Schichten aus gleichem Material gefertigt oder unterschiedlich ausgestaltet.

Gemäß verschiedener Ausführungsformen sind die Polymerbeschichtungen der äußeren Schichten und/oder die äußeren Schichten biostabil und/oder biodegradierbar und/oder mit Endothelzellen besiedelt.

Gemäß verschiedener Ausführungsformen weist zumindest die Polymerbeschichtung einer äußeren Schicht und/oder eine äußere Schicht eine Oberflächenmodifizierung und/oder eine Wirkstoffbeladung auf.

Gemäß verschiedener Ausführungsformen sind die äußeren Schichten in Form von Fasern und/oder Filmen und/oder Blends und/oder Folien und/oder Vliesen und/oder Kompositen ausgeführt.

Gemäß verschiedener Ausführungsformen ist das flexibel ausgestaltete Metallgebilde ein- oder mehrlagig.

Gemäß verschiedener Ausführungsformen ist das flexibel ausgestaltete Metallgebilde ein ultradünnes Gewebe und/oder Gewirk und/oder Geflecht und/oder Gelege und/oder Vlies und/oder Filz aus Drähten und/oder Fäden und/oder Filamenten und/oder Fasern und/oder Bändern und/oder Strängen und/oder Fibrillen.

Gemäß verschiedener Ausführungsformen weisen die Drähte und/oder Fäden und/oder Filamente und/oder Fasern und/oder Bänder und/oder Stränge und/oder Fibrillen eine Stärke im Mikrometerbereich auf.

Die Lösung der Aufgabe erfolgt ferner durch ein Verfahren zur Herstellung des Mehrschichtmaterials, wobei das Verfahren zur Herstellung des Mehrschichtmaterials folgende Verfahrensschritte aufweist:
a. Herstellung des Metallgebildes,
b. Reinigung des Metallgebildes,
c. Beschichtung des Metallgebildes.

Amorphe Metalle oder metallische Gläser sind Metall- oder Metall-und-Nichtmetall-Legierungen, welche auf atomarer Ebene keine kristalline, sondern eine amorphe Struktur aufweisen und trotzdem metallische Leitfähigkeit zeigen. Eine für Metalle sehr ungewöhnliche amorphe Atomanordnung hat eine einzigartige Kombination physikalischer Eigenschaften zur Folge: metallische Gläser sind im Allgemeinen härter, korrosionsbeständiger und fester als gewöhnliche Metalle.

Metallische Gläser sind härter als ihre kristallinen Gegenstücke und haben eine hohe Festigkeit. Geringe Verformungen (≈ 1 %) sind rein elastisch. Das heißt, aufgenommene Energie geht nicht als Verformungsenergie verloren, sondern wird beim Zurückfedern des Materials wieder voll abgegeben. Ihre Korrosionsbeständigkeit ist in der Regel höher als bei Metallen vergleichbarer chemischer Zusammensetzung. Dies liegt daran, dass Korrosion meist an Korngrenzen zwischen Einzelkristalliten eines Metalls angreift, die es bei amorphen Materialien nicht gibt.

Wie alle Gläser entstehen auch amorphe Metalle, indem eine natürliche Kristallisation verhindert wird. Dies kann zum Beispiel durch rasches Abkühlen ("Abschrecken") einer Schmelze geschehen, so dass den Atomen eine Beweglichkeit geraubt wird, bevor sie eine Kristallanordnung einnehmen können. Herkömmliche Metalle ziehen sich typischerweise beim Erstarren schlagartig zusammen. Da eine Erstarrung als Glas kein Phasenübergang erster Ordnung ist, findet dieser Volumensprung bei amorphen Metallen nicht statt. Wenn die Schmelze eines metallischen Glases eine Form ausfüllt, so behält sie diese beim Erstarren. Dies ist ein Verhalten, das man zum Beispiel von Polymeren kennt und große Vorteile bei einer Verarbeitung bietet.

Für den beschriebenen Erfindungsgegenstand kommen alle metallischen Gläser nach dem Stand der Technik in Betracht. Es existiert eine große Anzahl an Legierungen, welche in metallisches Glas gegossen werden können, wobei die beteiligten Elemente häufig kostengünstig sind. Eine entsprechende Auflistung liefert Greer 2009
(https://doi.org/10.1016/S1369-7021 (09)70037-9), wobei nicht ausgeschlossen ist, dass darüber hinaus weitere metallische Gläser Anwendung finden können.

Neben metallischem Glas kann das flexibel ausgestaltete Metallgebilde weitere Metalle und/oder Metall- und/oder Metall-und-Nichtmetall-Legierung aufweisen, beispielsweise aus Edelstahl und/oder Gold und/oder Silber und/oder Wolfram und/oder CoCr und/oder Titan und/oder Magnesium und/oder Nitinol. Ferner kann das flexibel ausgestaltete Metallgebilde Beimengungen von Seltenen Erden aufweisen.

Eine mechanische Belastbarkeit des Mehrschichtmaterials wird durch das flexibel ausgestaltete Metallgebilde in Form eines ultradünnen Gewebes und/oder Gewirks und/oder Geflechts und/oder Geleges und/oder Vlies und/oder Filzes aus Drähten und/oder Fäden und/oder Filamenten und/oder Fasern und/oder Bändern und/oder Strängen und/oder Fibrillen in Mikrometerdimension erreicht. Weitere beispielhafte Ausführungen von Strukturen zur Ausbildung des Metallgebildes stellen Hohlfasern, Verdrillungen, Komposite, Röhrchen, Nanoröhrchen, Koaxialfasern, - fäden, -filamente und ummantelte Fasern dar. Ultradünn bezieht sich hierbei auf eine Dicke, welche im Wesentlichen einer durchschnittlichen Dicke natürlicher Bikuspidalklappen und/oder Trikuspidalklappen und/oder Pulmonalklappen und/oder Aortenklappen entspricht.

Die Drähte und/oder Fäden und/oder Filamente und/oder Fasern und/oder Bänder und/oder Stränge und/oder Fibrillen des flexibel ausgestalteten Metallgebildes können einen beliebigen Querschnitt aufweisen. Fäden weisen typischerweise einen kreisrunden Querschnitt auf. Bänder weisen typischerweise einen rechteckigen Querschnitt auf. Typische Querschnitte können aber, beispielsweise durch geeignete Bearbeitungsverfahren, derart modifiziert werden, dass sie untypisch werden, beispielsweise in Form eines Dreiecks oder eines Ovals. Dies kann im Sinne einer beabsichtigen oder unbeabsichtigten Funktionalisierung erfolgen, beispielsweise um eine Biege- und/oder Bruchfestigkeit zu erhöhen.

Für die (Faser-)Beschichtung eignen sich alle bekannten biokompatiblen Polymere, naturfaserverstärkte Verbundwerkstoffe und High Performance Polymere, wobei vorzugsweise i) biostabile, inerte Polymere wie Polyfluorkohlenwasserstoffe (u.a. ePTFE), Polyurethane (PU), Polyamide (PA), Silikone, thermoplastische Polyester (TPE), einschließlich deren Co- oder Blockpolymere, Blendpartner oder Kompositsysteme und ii) biodegradierbare Polymere mit folgenden Monomereinheiten: Ester, Dioxanone, Milchsäure, Hydroxybuttersäure, Bernsteinsäure, Ethylenglycol, Cellulose(derivate), Chitosan, (Amino)Zucker, Zuckersäuren, Glykogen, Stärke, Lipide, die unabhängig von ihrer Rohstoffbasis (bio- oder erdölbasiert), Anwendung finden. Weitere für eine Beschichtung geeignete Polymere stellen Gelatine, bakterielle Cellulose, Alginat, Prokollagen, Kollagen, Hyaluronsäure, Glykoprotein, Fibronektin, Laminin, Muschel- und Seidenprotein, beispielsweise in Form von Hydrogelen, dar, wobei dem Fachmann bekannt ist, dass eine Vielzahl weiterer Polymere existieren, welche sich für eine erfindungsgemäße Beschichtung eignen.

Die erste äußere Schicht kann sich in ihrer Ausführung von der zweiten äußeren Schicht unterscheiden. Die erste Polymerbeschichtung muss demnach nicht zwingend identisch zur zweiten Polymerbeschichtung sein. Ferner kann das Mehrschichtmaterial eine Vielzahl äußerer Schichten aufweisen, welche aus verschiedenen Polymeren gebildet sein können. Beispielsweise kann eine Seite des Mehrschichtmaterials eine äußere Schicht aufweisen, während die andere Seite des Mehrschichtmaterials drei äußere Schichten aufweist.

Ein Aufbringen der Polymerbeschichtungen auf das flexibel ausgestaltetes Metallgebilde kann beispielsweise mittels Elektrospinning, Elektrospraying, Tauchbeschichtung, Sprühbeschichtung, Aufpressen, Walzen, Schlagen, Punzieren, und/oder Schweißen erfolgen. Weitere Methoden, welche ein Aufbringen einer Polymer- oder ähnlichen Beschichtung auf das Metallgebilde ermöglichen sind denkbar.

Eine Verbindung einzelner Schichten kann während des Auftragens oder in einem separaten Prozessschritt erfolgen. Eine ausreichende Haftfestigkeit oder Schichtintegrität kann über verschiedene in-situ Verfahren zur Fasererzeugung gewährleistet werden, indem u.a. über eine Anpassung der Lösungsmittelmenge oder des Elektrodenabstandes ein Übergang vom Elektrospraying zum Elektrospinning erfolgt.

Es können weitere post-prozessive Verfahren zur Hybridisierung von Metallgebilde und Polymerbeschichtung zum Mehrschichtmaterial genutzt werden, beispielsweise eine Mikrostrukturierung durch u.a. Ultraschallschweißen oder Laserprozesse sowie thermische, (nass-) chemische, mechanische oder Kombinationsverfahren unter Nutzung von Bedampfung oder klebstoffbasierten Fügeverfahren. Ebenfalls denkbar sind strahlenbasierte post-prozessive Verfahren, beispielsweise eine Strahlenvernetzung (β, γ) und/oder UV-Vernetzung.

Beidseitig aufgebrachte und in geeigneter Weise befestigte polymere Beschichtungen, wie beispielsweise Nanofaservliese oder Dünnfilme, sorgen für eine nötige Bio- und Hämokompatibilität bei medizinischen Anwendungen, insbesondere bei einer Verwendung des Mehrschichtmaterials als medizinisches Implantat. Eine Funktion polymerer Faservliese im Verbund mit dem Metallgebilde liegt zum einen in einer Verbesserung einer zellulären Besiedelung des Mehrschichtmaterials durch Mimikry einer natürlichen Faserstruktur einer Extrazellulärmatrix mit Faserdurchmessern im Submikrometerbereich (50 nm - 5 µm), und zum anderen in einer Verminderung einer Blutpermeabilität.

Hierbei kann eine Beschichtung in ihren Ausführungen sowohl dauerhaft als auch bioresorbierbar sein. Die Hämokompatibilität kann darüber hinaus mittels Tissue Engineering unterstützt werden, indem eine vorherige Endothelzellbesiedelung stattfindet.

Ferner können zur Erreichung einer gewünschten Biokompatibilität eine Oberflächenmodifizierung oder eine Wirkstoffbeladung zumindest einer der äußeren Schichten genutzt werden, sodass das Mehrschichtmaterial als Drug Delivery System (DDS) genutzt werden kann. Hierzu können beispielsweise verschiedene Atmopshären- oder Niederdruck-Plasmaakivierungen, unter anderen mit Luft, Ammoniak, Sauerstoff oder Stickstoff, oder eine chemische Anbindung spezieller Substanzen, wie aktiver Proteine über Crosslinkerchemie, genauso wie die Inkorporation von aktiven pharmazeutischen Wirkstoffen (API) in die polymeren Beschichtungen vorgenommen werden.

Die Lösung der Aufgabe erfolgt ferner durch eine Anwendung des Mehrschichtmaterials, wobei die Anwendung des Mehrschichtmaterials als Filter, Ventilbauteil oder temporäre und/oder permanente Implantatanwendung bei Knochendefekten erfolgt.

Anwendungen des Mehrschichtmaterials über medizinische Applikationen hinaus sind denkbar. Hierbei kann das flexibel ausgestaltetes Metallgebilde sowie die erste und zweite Polymerbeschichtung entsprechend eines Anforderungsprofils einer Applikation gewählt werden. Beispielsweise kann das flexibel ausgestaltete Metallgebilde eine mechanische Stabilität unter hoher Beanspruchung (Druck, Durchsatz), beispielsweise während einen Pump- oder Filtrationsvorgangs mittels des Mehrschichtmaterials, ermöglichen. Die Beschichtungen können mit unterschiedlicher Porengröße ausgeformt sein, um beispielsweise einen zweistufigen Filter für einen Partikel-Filtervorgang bereitzustellen. Ferner können die Polymerbeschichtungen chemisch derart modifiziert sein, dass sie als selektive lonenfilter fungieren. Aufgrund einer Vielzahl an Individualisierungsmöglichen in Bezug auf die Ausgestaltung der zumindest zwei äußeren Schichten und der zumindest einen inneren Schicht sind eine Vielzahl weiterer Applikationen denkbar.

Die Lösung der Aufgabe erfolgt ferner durch ein medizinisches kardiovaskuläres Implantat, wobei das medizinische kardiovaskuläre Implantat das Mehrschichtmaterial aufweist. Das Mehrschichtmaterial weist eine Segelform und zumindest eine vordefinierte fixierte Falte auf, um eine mechanische und strömungsdynamische Stabilität des medizinischen Implantates zu erreichen.

Gemäß verschiedener Ausführungsformen ist die zumindest eine vordefinierte fixierte Falte asymmetrisch gebogen, um Totbereiche stagnierender Strömung zu vermeiden.

Gemäß verschiedener Ausführungsformen ist die Fixierung der zumindest einen vordefinierten Falte formschlüssig und/oder kraftschlüssig und/oder stoffschlüssig.

Gemäß verschiedener Ausführungsformen ist das medizinische kardiovaskuläre Implantat ein Okkluder oder eine Herzklappenprothese.

Gemäß verschiedener Ausführungsformen ist das medizinische kardiovaskuläre Implantat eine Einsegelklappe.

Gemäß verschiedener Ausführungsformen ist die Einsegelklappe zur offenchirurgischen und Transkatheter-Aortenklappenimplantation vorgesehen.

Die Lösung der Aufgabe erfolgt ferner durch ein Verfahren zur Herstellung des medizinischen kardiovaskulären Implantats, wobei das Verfahren zur Herstellung des medizinischen kardiovaskulären Implantats folgende Verfahrensschritte aufweist:
a. Herstellung eines Mehrschichtmaterials nach Anspruch 1 durch
   i. Herstellung des Metallgebildes,
   ii. Reinigung des Metallgebildes,
   iii. Beschichtung des Metallgebildes,
b. Zuschnitt des Mehrschichtmaterials,
c. Erzeugen zumindest einer vordefinierten fixierten Falte im Mehrschichtmaterial durch Legung der Falte, Fixierung der Falte und Erzeugung einer Vorbiegung der Falte.

Der Zuschnitt des Metallgebildes kann vor oder nach der Reinigung des Metallgebildet erfolgen. Ferner kann der Zuschnitt des Metallgebildes vor oder nach der Beschichtung des Metallgebildes erfolgen.

Die Fixierung der zumindest einen vordefinierten fixierten Falte erfolgt beispielsweise per Naht mit Draht oder anderen etablierten Nahtmaterialien, bzw. mittels Schweißverfahren, Klebeverfahren, Klammerverfahren, Steck- bzw. Klemmverfahren, oder kombinierten Verfahren.

Gemäß verschiedener Ausführungsformen erfolgt die Erzeugung der zumindest einen vordefinierten Falte, indem das Mehrschichtmaterial geknickt, fixiert und nach einer Seite vorgebogen wird.

Die neuartige Segelform des medizinischen kardiovaskulären Implantats ist gekennzeichnet durch ein oder mehrere vordefinierte fixierte Falten, welche asymmetrisch gebogen sein können und dem Segel mechanische und strömungsdynamische Stabilität verleihen.

Das Segel ist bei jedem Zyklus, d.h. bei jedem Öffnungs- und/oder Schließvorgang, nur geringeren lokalen Verformungen bzw. Krümmungsänderungen ausgesetzt. Somit gibt es keine Bereiche, welche wiederholt hohen Lastspitzen ausgesetzt sind. Dies verlängert die Lebensdauer des medizinischen kardiovaskulären Implantats.

Beim Schließen herkömmlicher mechanischer Klappen auftretende Klickgeräusche, die von vielen Patienten als störend empfunden werden, treten bei dem erfindungsgemäßen medizinischen kardiovaskulären Implantat unter Verwendung des Mehrschichtmaterials nicht auf.

Das medizinische kardiovaskuläre Implantat kann problemlos an nicht-kreisrunde Querschnitte adaptiert und in patientenindividuell geformte Haltesysteme wie Stents oder Ringe eingepasst werden.

Eine Befestigung des medizinischen kardiovaskulären Implantats im Haltesystem (z.B. Stent) kann beispielsweise per Naht mit Draht oder anderen etablierten Nahtmaterialien, bzw. mittels Schweißverfahren, Klebeverfahren, Klammerverfahren, Steck- bzw. Klemmverfahren, oder kombinierten Verfahren erfolgen.

Hierbei wird das flexibel ausgestaltete Mehrschichtmaterial durch einen Crimpprozess, der für eine katheterbasierte Implantation des medizinischen kardiovaskulären Implantats notwendig ist, nicht beschädigt oder in seiner Funktion beeinträchtigt.

Ferner bleibt bei geeigneter Beschichtung eine potenzielle Kalzifizierung auf den zwei äußeren Schichten begrenzt, sodass die mechanischen Eigenschaften nicht beeinflusst werden, was eine Betriebsdauer des erfindungsgemäßen medizinischen kardiovaskulären Implantats erhöht. Im Vergleich hierzu können Klappen aus biologischem Material durch Kalzifizierung verhärten und in ihrer Funktion nachhaltig beeinträchtigt werden.

Eine problemlose Valve-in-Valve-Implantation des erfindungsgemäßen medizinischen kardiovaskulären Implantats ist im Falle einer notwendigen Revision möglich.

Das erfindungsgemäße medizinische kardiovaskuläre Implantat erlaubt Ausführungsformen mit unterschiedlicher Segelanzahl. So können beispielsweise statt der üblichen tricuspiden Form auch zwei oder ein Segel verwendet werden. Bei Verwendung mehrerer Segel können diese nach Bedarf unterschiedlich groß gestaltet werden, sodass jedes Segel zu einem unterschiedlichen Anteil an der Öffnungsfläche einer Klappenprothese beiträgt.

Durch die Faltenlegung im Mehrschichtmaterial bzw. in dem Segel / den Segeln kann u.a. eine Anpassung an wechselnde Längen und Orientierungen einer Segeloberkante während eines Öffnens/Schließens der Klappe (etwa halber Umfang↔Durchmesser↔halber Umfang) gewährleistet werden, ohne die lokale Wölbung des Materials wesentlich zu ändern. Mittels eines sogenannten Ziehharmonikaprinzips, welches darauf basiert, dass ein relativ steifes Material in flexibler Zickzack-Anordnung (Falten), verschiedene Randabstände und Gesamtverformungen mit wenig Kraftaufwand zulässt kann die Überschuss-Länge unter anderem im Falle einer Klappenprothese mit nur einem Segel dabei in den Falten bei lokal nur geringer Verformung "verstaut" werden.

Ein zügiger Schließvorgang wird durch eine elastische Rückstellung des Segels in einem teilgeöffneten Zustand (ohne äußere Kräfte) begünstigt.

Dabei liegt die Segeloberkante im geschlossenen Zustand an der Außenwand des Haltesystems (z.B. Stent) an und es verbleibt ein sehr geringes Restlumen im Bereich der Faltenspitze(n). Zudem wird durch die asymmetrische(n) Falte(n) beim Schließvorgang die sich bildende Tasche durchströmt, sodass Totbereiche stagnierender Strömung vermieden werden (Stagnation begünstigt die Bildung von Blutgerinnseln und Thrombosen).

Beim Öffnungsvorgang legt/legen sich die Falte(n) aufgrund der Vorkrümmung ohne punktuelle Belastung sanft an die Außenwand des Haltesystems (z.B. Stent) an.

Die Neuartigkeit der Erfindung bezieht sich im Wesentlichen auf zwei Aspekte: (i) ein neuartiges metallgewebeverstärktes Mehrschichtmaterial und (ii) einer darauf abgestimmten gefalteten Segelform.

Die Erfindung ist eine Kombination aus einem mechanisch langzeitstabilen Mehrschichtmaterial mit zellbesiedlungsfreundlicher Oberfläche, und einer Segelform, die im Sinne eines werkstoffgerechten Designs, trotz einer Steifigkeit des Materials, durch eine Faltenlegung leicht beweglich ist und mechanisch dem biologischen Vorbild ähnelt. Die Erfindung ist grundsätzlich für offenchirurgische als auch minimalinvasive Implantationstechniken geeignet.

Dem ersten Aspekt der Erfindung liegt die Überlegung zugrunde, dass ein langzeitbelastbares flexibles Material, dass bereitwillig von Endothelzellen besiedelt wird, bislang nicht bekannt ist. Daher werden die gewünschten Eigenschaften an geeignete Materialen adressiert, die in ihrer Gesamtheit als Schichtverbund die gewünschten Eigenschaften vereinen.

Dem zweiten Aspekt der Erfindung liegt die Überlegung zugrunde, dass die Einzeldrähte eines Metallgebildes mit Durchmessern im Mikrometerbereich bei gleicher makroskopischer Verformung, beispielsweise beim Öffnungs- und Schließvorgang von Segelklappen, einen geringen relativen Biegeradius erfahren. Dabei werden die Drähte weit unterhalb der Dehngrenze belastet (elastisch) und nicht in den Bereich der plastischen Verformung oder Bruchbelastung gebracht. In Analogie zu flexiblen Glasfaserkabeln, die als Faser von Haaresbreite Biegeradien im Zentimeterbereich unbeschadet überstehen, bricht Glas von makroskopischen Abmessungen bereits bei geringer Verformung.

Die vorliegende Erfindung resultiert in der Vermeidung der Xenotransplantation tierischer Gewebebestandteile (u.a. Perikard), was unter anderem in Anbetracht des benötigten Aufwands für die Aufbereitung, aber auch in Anbetracht ethischer Aspekte vorteilhaft ist.

Der Erfindungsgegenstand lässt sich mittels reproduzierbarer Herstellungsprozesse fertigen, und weist somit im Vergleich zu biologischem Material, bei einer gleichzeitig verringerten Anzahl an Prozessschritten, gleichbleibende Materialeigenschaften aus. Dies erlaubt die Etablierung von Maßnahmen zur Sicherstellung festgelegter Qualitätsanforderungen im Sinne einer Qualitätssicherung.

Ferner unterliegen die eingesetzten Materialien keiner nennenswerten Materialermüdung, sodass ein konstantes Betriebsverhalten über die gesamte Lebensdauer gesichert ist.

Im Vergleich zu Xenotransplantaten ist die Lebensdauer signifikant erhöht.

Im Vergleich zu Transplantaten gemäß dem Stand der Technik sind die Herstellungskosten signifikant reduziert.

Neben der Herzklappenprothese sind weitere potenzielle Einsatzfelder kardiovaskulärer Anwendungen z.B. als Okkluder, Klappenstrukturen im Bereich des Venen- oder Lymphgefäßsystems sowie temporäre oder permanente Implantatanwendungen, z.B. bei Knochendefekten der Kalotte, denkbar.

Darüber hinaus zeichnet sich der Erfindungsgegenstand durch seine Anpassungsfähigkeit aus. So kann dieser beispielsweise individuell für einen bestimmten Patienten hinsichtlich Materialkombination, Segelform und Stentdesign adaptiert werden, auch bei anatomisch schwierigem Situs.

Ferner kann der Erfindungsgegenstand an nicht-kreisrunde Querschnitte, oder an patientenindividuelle anatomische Gegebenheiten angepasst werden. Er hat somit Potential zur Verringerung von Schäden am Erregungsleitungssystem und paravalvulären Leckagen beizutragen.

Durch vielfältige Möglichkeiten der Materialkomposition, gezielt zugeschnitten für die jeweilige Anwendung, stellt der Erfindungsgegenstand eine Plattformtechnologie für eine Vielzahl von Anwendungsfeldern dar.

Die gefaltete Segelform lässt sich anwendungsübergreifend im Bereich Förder- und Pumpsysteme, Ventil- bzw. Klappentechnik nutzen.

So eignet sich der Erfindungsgegenstand grundsätzlich auch als Ventil für technische Anwendungen, z.B. für Medien in Rohren, Industrie, Hochtemperaturprozesse, Rückschlagventile, nicht-newtonsche Fluide, Abfüllanlagen, verringerter Druckschlag beim Schließen, Sicherheitsventil.

Hierbei können Ein- oder Mehrsegelkombinationen gezielt für die jeweilige Strömungssituation zugeschnitten werden.

Das Mehrschichtmaterial bietet Potenzial als Filter für Spezialanwendungen unter mechanisch hochbeanspruchenden Bedingungen.

### Ausführung der Erfindung

Die Erfindung wird anhand eines/mehrerer Ausführungsbeispiels näher erläutert. Hierzu zeigen
- Figur 1: schematische Darstellung des Gesamtkonzepts,
- Figur 2A: Aufbau Ausführungsbeispiel Einsegelklappe mit einer Falte; Ansicht von der Seite und von Ausstromseite geschlossen,
- Figur 2B: Aufbau Ausführungsbeispiel Einsegelklappe mit einer Falte; Ansicht von der Seite und von Ausstromseite halbgeöffnet,
- Figur 2C: Aufbau Ausführungsbeispiel Einsegelklappe mit einer Falte; Ansicht von vorn,
- Figur 3: Aufbau Ausführungsbeispiel Zweisegelklappe mit einer Falte (oberes Segel) und zwei Falten (unteres Segel) im ovalen Querschnittsprofil; Ansicht von der Seite und von Ausstromseite halbgeöffnet.

In der Beschreibung wird auf die beigefügten Zeichnungen Bezug genommen, in denen zur Veranschaulichung spezifische Ausführungsformen gezeigt sind, in denen die erfindungsgemäße Anordnung ausgeübt werden kann. In dieser Hinsicht wird eine Richtungsterminologie wie etwa "oben", "unten" usw. mit Bezug auf die Orientierung der beschriebenen Zeichnungen verwendet. Die Richtungsterminologie dient der Veranschaulichung und ist auf keinerlei Weise einschränkend.

Es versteht sich, dass andere Ausführungsformen benutzt und strukturelle oder logische Änderungen vorgenommen werden können, ohne von dem Schutzumfang der vorliegenden Erfindung abzuweichen. Es versteht sich, dass die Merkmale der hierin beschriebenen verschiedenen beispielhaften Ausführungsformen miteinander kombiniert werden können, sofern nicht spezifisch anders angegeben. Die folgende ausführliche Beschreibung ist deshalb nicht in einschränkendem Sinne aufzufassen, und der Schutzumfang der vorliegenden Erfindung wird durch die angefügten Ansprüche definiert.

In den Figuren werden identische oder ähnliche Elemente mit identischen Bezugszeichen versehen, soweit dies zweckmäßig ist.

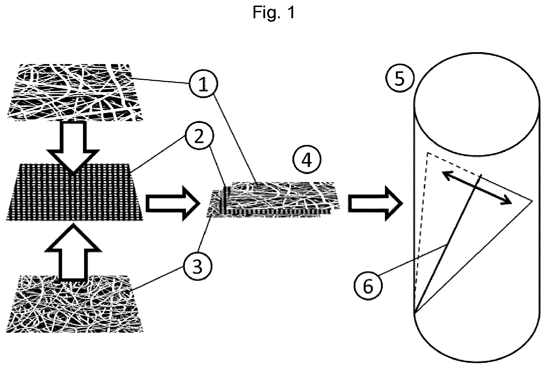

Das erfindungsgemäße Mehrschichtmaterial 4 ist in Figur 1 dargestellt. Das Mehrschichtmaterial 4 weist zumindest zwei äußere Schichten 1,3 und zumindest eine innere Schicht 2 auf. Die erste äußere Schicht 1 umfasst eine erste Polymerbeschichtung. Die zweite äußere Schicht 3 umfasst eine zweite Polymerbeschichtung. Die innere Schicht 2 umfasst ein flexibel ausgestaltetes Metallgebilde, welches metallisches Glas aufweist. Die Schichten 1,2,3 sind mittels unlösbarer Verbindungen miteinander fixiert, wobei die innere Schicht 2 zwischen der ersten äußeren Schicht 1 und der zweiten äußeren Schicht 3 angeordnet ist. Das Mehrschichtmaterial 4 kann eine vordefinierte fixierte Falte 7 aufweisen, hier nicht gezeigt. Die Polymerbeschichtungen der äußeren Schichten und/oder die äußeren Schichten 1,3 können aus gleichem Material gefertigt oder unterschiedlich ausgestaltet sein. Gemäß Figur 1 sind die äußeren Schichten 1,3, welche in Form von Fasern und/oder Filmen und/oder Blends und/oder Folien und/oder Vliesen und/oder Kompositen ausgeführt sein können, unterschiedlich ausgestaltet, wobei die erste äußere Schicht 1 beispielsweise eine höhere Porengröße als die zweite äußere Schicht 3 aufweist. Eine oder mehrere der äußeren Schichten 1,3 können biostabil und/oder biodegradierbar und/oder mit Endothelzellen besiedelt sein und/oder eine Oberflächenmodifizierung und/oder eine Wirkstoffbeladung aufweisen. Das flexibel ausgestaltete Metallgebilde der zumindest einen inneren Schicht 2 kann ein- oder mehrlagig ausgebildet sein und ein ultradünnes Gewebe und/oder Gewirk und/oder Geflecht und/oder Gelege und/oder Vlies und/oder Filz aus Drähten und/oder Fäden und/oder Filamenten und/oder Fasern und/oder Bändern und/oder Strängen und/oder Fibrillen darstellen, wobei die Drähte und/oder Fäden und/oder Filamente und/oder Fasern und/oder Bänder und/oder Stränge und/oder Fibrillen eine Stärke im Mikrometerbereich aufweisen. Gemäß Figur 1 ist das flexibel ausgestaltete Metallgebilde der zumindest einen inneren Schicht 2 als Gewebe ausgestaltet.

Das erfindungsgemäße Verfahren zur Herstellung des Mehrschichtmaterials 4 ist ebenfalls schematisch in Figur 1 dargestellt. Das Verfahren umfasst die Herstellung des Metallgebildes als innere Schicht 2, die Reinigung des Metallgebildes und die Beschichtung des Metallgebildes mit, hier mit einer ersten äußeren Schicht 1 und einer zweiten äußeren Schicht 3.

Ebenfalls in Figur 1 dargestellt ist das erfindungsgemäße medizinische kardiovaskuläre Implantat, welches das Mehrschichtmaterial 4 aufweist, und hier in Figur 1 als bewegliche Segelklappe in einem Gefäß 5 eingesetzt ist. Das Mehrschichtmaterial 4 weist eine Segelform und zumindest eine vordefinierte fixierte Falte 7 auf (hier nicht gezeigt), um eine mechanische und strömungsdynamische Stabilität des medizinischen Implantates zu erreichen. Das erfindungsgemäße medizinische kardiovaskuläre Implantat kann, wie in Figur 1 gezeigt, mittels einer Befestigung 6 an einer Außenwand einer Haltekonstruktion 9 (z.B. Stent, hier nicht gezeigt) fixiert werden.

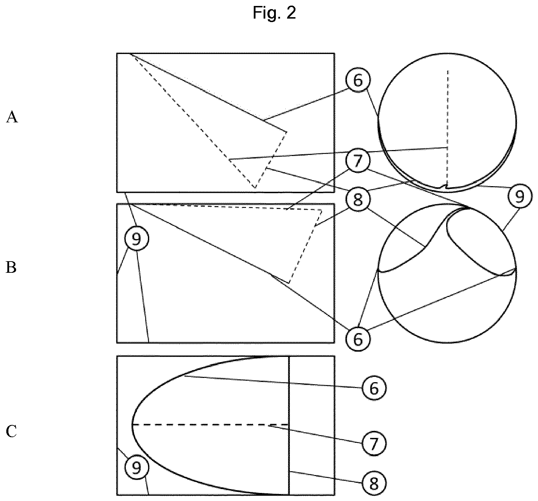

Figur 2 zeigt ein mögliches Ausführungsbeispiel des erfindungsgemäßen medizinischen kardiovaskulären Implantats, wobei das medizinische kardiovaskuläre Implantat bevorzugt als Einsegelklappe ausgeführt ist. Wie in Figur 2, im Speziellen in Figur 2B, gezeigt, ist ein einer vorteilhaften Ausgestaltung die zumindest eine vordefinierte fixierte Falte 7 asymmetrisch gebogen, um Totbereiche stagnierender Strömung zu vermeiden. Hierbei kann die Fixierung der zumindest einen vordefinierten Falte 7 formschlüssig und/oder kraftschlüssig und/oder stoffschlüssig erfolgen, wie später in Anwendungsbeispielen beispielhaft erläutert wird. Gemäß Figur 2 findet das medizinische kardiovaskuläre Implantat in Form einer Einsegelklappe in einer Haltekonstruktion 9, beispielsweise einem Stent, Anwendung. Dort kann es mittels einer Befestigung 6 an einer Außenwand fixiert werden. Im geschlossenen Zustand (Figur 2A) liegt eine Segeloberkante 8 eng an der Außenwand der Haltekonstruktion 9 an, und es verbleibt lediglich ein sehr geringes Restlumen im Bereich der Spitze der vordefinierten fixierten Falte 7. Das Gefäß 5 (hier nicht gezeigt) ist demnach im Wesentlichen geschlossen. Im halboffenen Zustand befindet sich die Segeloberkante 8 nahe der Außenwand der Haltekonstruktion 9, an der das medizinische kardiovaskuläre Implantat befestigt ist. Beim Öffnungsvorgang legt sich die vordefinierte fixierte Falte 7 aufgrund der Vorkrümmung ohne punktuelle Belastung sanft an die Außenwand der Haltekonstruktion 9 an und schon diese hiermit. Das medizinische kardiovaskuläre Implantat kann ein Okkluder oder eine Herzklappenprothese sein und kann mittels offenchirurgischer oder Transkatheter-Aortenklappenimplantation implantiert werden.

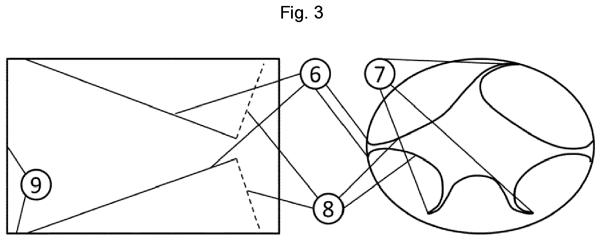

Figur 3 zeigt ein weiteres mögliches Ausführungsbeispiel des erfindungsgemäßen medizinischen kardiovaskulären Implantats, wobei das medizinische kardiovaskuläre Implantat hier als Zweisegelklappe ausgeführt ist. Gemäß Figur 3 findet das medizinische kardiovaskuläre Implantat in Form einer Zweisegelklappe in einer Haltekonstruktion 9, beispielsweise einem Stent, Anwendung. Hierbei weist die Haltekonstruktion 9 einen ovalen Querschnitt auf, beispielsweise als patientenindividuelle Anpassung an ein Gefäß 6 (hier nicht gezeigt). An der ovalen Haltekonstruktion 9 kann jedes der zwei Segel mittels einer separaten Befestigung 6 an einer Außenwand fixiert werden. Wie in Figur 3 ersichtlich, weist das obere Segel eine vordefinierte fixierte Falte 7 auf, während das untere Segel zwei vordefinierte Falten 7 aufweist. Im dargestellten halboffenen Zustand befinden sich die Segeloberkanten 8 nahe der Außenwand der Haltekonstruktion 9, an der das medizinische kardiovaskuläre Implantat befestigt ist. Beim Öffnungsvorgang legen sich die vordefinierten fixierten Falten 7 aufgrund der Vorkrümmung ohne punktuelle Belastung sanft an die Außenwand der Haltekonstruktion 9 an und schonen diese hiermit.

Im Folgenden wird die Erfindung anhand mehrerer Anwendungsbeispiele erläutert.

### Anwendungsbeispiel 1: Einsegelklappe im Stent mit einer Falte, Lösung1 + Vlies2 legen

Das Metallgebilde wird nach intensiver Reinigung bei Bedarf mit einer Haftvermittlerlösung versehen und direkt im Anschluss einseitig mit PLLA-Lösung beschichtet. Die andere Seite wird in einem thermischen Prozessschritt mit PCL-Polymerfaservlies verpresst und derart in einem Mehrschichtmaterial vereint, dass sich dessen Bestandteile (Schichten) nicht mehr voneinander lösen. Das erzeugte Mehrschichtmaterial wird in passender Größe zugeschnitten. Die Falte wird erzeugt, indem das Material zunächst geknickt, danach mittels Klammerung fixiert, und nach einer Seite vorgebogen wird. Durch die Fixierung wird eine vordefinierte Wölbung erreicht, die dauerhaft erhalten bleibt und ein gleichmäßiges Betriebsverhalten erlaubt. Durch die Vorbiegung wird verhindert, dass die Kante der Falte direkt auf die Außenwand des Stents trifft und Beschädigungen auftreten. Die Außenkante des Segels wird schräg im Nitinol-Stent an den Streben per Naht befestigt, sodass beim Öffnen/Schließen die freie Oberkante problemlos die Außenwände des Stents erreichen kann (Figuren 1,2).

### Anwendungsbeispiel 2: Einsegelklappe im Stent mit einer Falte, Vlies1+2 legen

Das Metallgebilde wird nach der Reinigung/Vorbehandlung einseitig mit PLLA-Polymerfaservlies belegt. Die zweite Seite wird mit PCL-Polymerfaservlies belegt. In einem thermischen Prozessschritt wird durch Zusammenpressen ein Mehrschichtmaterial erzeugt, dessen Bestandteile (Schichten) sich nicht mehr voneinander lösen. Das Mehrschichtmaterial wird in passender Größe zugeschnitten. Die Falte wird zunächst geknickt, danach mittels Naht fixiert, und nach einer Seite vorgebogen. Durch die Fixierung wird eine vordefinierte Wölbung erreicht, die dauerhaft erhalten bleibt und ein gleichmäßiges Betriebsverhalten erlaubt. Durch die Vorbiegung wird verhindert, dass die Kante der Falte direkt auf die Außenwand des Stents trifft und Beschädigungen auftreten. Die Außenkante des Segels wird schräg im Nitinol-Stent an den Streben per Schweißung befestigt, sodass beim Öffnen/Schließen die freie Oberkante problemlos die Außenwände des Stents erreichen kann (Figuren 1,2).

### Anwendungsbeispiel 3: Einsegelklappe im Stent mit einer Falte, Vlies1 spinnen + Folie2 legen

Das Metallgebilde wird nach intensiver Reinigung einseitig mit TSPCU-Polymerfaservlies elektrobesponnen. Die andere Seite wird mit Hytrel-Folie belegt. In einem Prozessschritt werden die Polymere mittels Lösungsmitteldampf (Chloroform) oberflächlich erweicht/angelöst und somit verklebt zu einem Mehrschichtmaterial, dessen Bestandteile (Schichten) sich nicht mehr voneinander lösen. Das erzeugte Mehrschichtmaterial wird in passender Größe zugeschnitten. Die Falte wird erzeugt, indem das Material zunächst geknickt, danach mittels Verklebung fixiert, und nach einer Seite vorgebogen wird. Durch die Fixierung wird eine vordefinierte Wölbung erreicht, die dauerhaft erhalten bleibt und ein gleichmäßiges Betriebsverhalten erlaubt. Durch die Vorbiegung wird verhindert, dass die Kante der Falte direkt auf die Außenwand des Stents trifft und Beschädigungen auftreten. Die Außenkante des Segels wird schräg im CoCr-Stent an den Streben per Naht befestigt, sodass beim Öffnen/Schließen die freie Oberkante problemlos die Außenwände des Stents erreichen kann (Figuren 1,2).

### Anwendungsbeispiel 4: Einsegelklappe im Stent mit einer Falte, Vlies1 spinnen + Vlies2 spinnen

Das Metallgebilde wird nach intensiver Reinigung einseitig mit PLLA/Gelatine-Polymerfaservlies elektrobesponnen. Die andere Seite wird mit Polydioxanon-Polymerfaservlies elektrobesponnen. In einem Prozessschritt wird das Zwischenerzeugnis mit Lösungsmittel benebelt (TFE), somit oberflächlich erweicht/angelöst und durch mechanisches Pressen zu einem Mehrschichtmaterial verklebt, dessen Bestandteile (Schichten) sich nicht mehr voneinander lösen. Das erzeugte Mehrschichtmaterial wird in passender Größe zugeschnitten. Die Falte wird erzeugt, indem das Material zunächst geknickt, danach mittels Verklebung fixiert, und nach einer Seite vorgebogen wird. Durch die Fixierung wird eine vordefinierte Wölbung erreicht, die dauerhaft erhalten bleibt und ein gleichmäßiges Betriebsverhalten erlaubt. Durch die Vorbiegung wird verhindert, dass die Kante der Falte direkt auf die Außenwand des Stents trifft und Beschädigungen auftreten. Die Außenkante des Segels wird schräg im CoCr-Stent an den Streben per Verklebung befestigt, sodass beim Öffnen/Schließen die freie Oberkante problemlos die Außenwände des Stents erreichen kann (Figuren 1,2).

### Anwendungsbeispiel 5: Einsegelklappe im Stent mit einer Falte, Folie1+2 legen

Das Metallgebilde wird nach intensiver Reinigung und Beschichtung mit Silikonkleber beidseitig mit Silikon-Folie belegt. In einem Prozessschritt wird durch mechanisches Pressen ein Mehrschichtmaterial verklebt, dessen Bestandteile (Schichten) sich nicht mehr voneinander lösen. Das erzeugte Mehrschichtmaterial wird in passender Größe zugeschnitten. Die Falte wird erzeugt, indem das Material zunächst geknickt, danach mittels Verklebung fixiert, und nach einer Seite vorgebogen wird. Durch die Fixierung wird eine vordefinierte Wölbung erreicht, die dauerhaft erhalten bleibt und ein gleichmäßiges Betriebsverhalten erlaubt. Durch die Vorbiegung wird verhindert, dass die Kante der Falte direkt auf die Außenwand des Stents trifft und Beschädigungen auftreten. Die Außenkante des Segels wird schräg im Nitinol-Stent an den Streben per Naht befestigt, sodass beim Öffnen/Schließen die freie Oberkante problemlos die Außenwände des Stents erreichen kann (Figuren 1,2).

### Anwendungsbeispiel 6: Zweisegelklappe im anatomischen Stent mit einer bzw. zwei Falten, Lösung 1+ Vlies2 spinnen

Das Metallgebilde wird nach intensiver Reinigung einseitig mit Hytrel-Lösung beschichtet. Die andere Seite wird mit PLLA -Polymerfaservlies elektrobesponnen. In einem Prozessschritt wird das Zwischenerzeugnis mit Lösungsmittel benebelt (TFE), somit oberflächlich erweicht/angelöst und durch mechanisches Pressen zu einem Mehrschichtmaterial verklebt, dessen Bestandteile (Schichten) sich nicht mehr voneinander lösen. Das erzeugte Mehrschichtmaterial wird in passender Größe zugeschnitten. Die Falte in Segel 1 wird erzeugt, indem das Material zunächst geknickt, danach mittels Naht fixiert, und nach einer Seite vorgebogen wird. In Segel 2 werden analog zwei Falten gelegt und jeweils nach außen vorgebogen. Durch die Fixierung wird eine vordefinierte Wölbung erreicht, die dauerhaft erhalten bleibt und ein gleichmäßiges Betriebsverhalten erlaubt. Durch die Vorbiegung wird verhindert, dass die Kante der Falte direkt auf die Außenwand des Stents trifft und Beschädigungen auftreten. Die Außenkanten der Segel werden schräg in einen CoCr-Stent, der an die individuelle Anatomie des Patienten adaptiert ist, per Verschweißung befestigt. Beim Öffnen/Schließen ermöglichen die freien Oberkanten eine problemlose Koaptation bzw. die Berührung der Außenwände des Stents (Figuren 1,3).

### Anwendungsbeispiel 7: Filteranwendung

Das Metallgebilde wird nach intensiver Reinigung einseitig mit TSPCU-Polymerfaservlies elektrobesponnen. Die andere Seite wird mit Nylon-Polymerfaservlies elektrobesponnen. In einem Prozessschritt wird durch thermisch-mechanisches Pressen ein Mehrschichtmaterial erzeugt, dessen Bestandteile (Schichten) sich nicht mehr voneinander lösen. Die verdichteten Vlieslagen haben eine unterschiedliche Porengröße und stellen damit einen zweistufigen Filter dar. Das metallische Stützgewebe sorgt für mechanische Stabilität, was einen Einsatz unter hoher Beanspruchung (Druck, Durchsatz) ermöglicht.

### Bezugszeichen

- 1: erste äußere Schicht
- 2: innere Schicht
- 3: zweite äußere Schicht
- 4: Mehrschichtmaterial
- 5: Gefäß
- 6: Befestigung an Außenwand der Haltekonstruktion
- 7: vordefinierte fixierte Falte
- 8: Segeloberkante
- 9: Haltekonstruktion

## Patentansprüche

1. Mehrschichtmaterial (4), aufweisend
zumindest zwei äußere Schichten (1,3) und zumindest eine innere Schicht (2),
wobei die erste äußere Schicht (1) eine erste Polymerbeschichtung umfasst und die zweite äußere Schicht (3) eine zweite Polymerbeschichtung umfasst, wobei
die innere Schicht (2) ein flexibel ausgestaltetes Metallgebilde umfasst, wobei
das flexibel ausgestaltete Metallgebilde metallisches Glas aufweist, und wobei
die Schichten (1,2,3) mittels unlösbarer Verbindungen miteinander fixiert sind, derart, dass die innere Schicht (2) zwischen der ersten äußeren Schicht (1) und der zweiten äußeren Schicht (3) angeordnet ist.

2. Mehrschichtmaterial (4) gemäß Anspruch 1, ferner aufweisend zumindest eine vordefinierte fixierte Falte (7).

3. Mehrschichtmaterial (4) gemäß einem der vorhergehenden Ansprüche,
**dadurch gekennzeichnet, dass** die Polymerbeschichtungen der äußeren Schichten und/oder die äußeren Schichten (1,3) aus gleichem Material gefertigt oder unterschiedlich ausgestaltet sind.

4. Mehrschichtmaterial (4) gemäß einem der vorhergehenden Ansprüche,
**dadurch gekennzeichnet, dass** die Polymerbeschichtungen der äußeren Schichten und/oder die äußeren Schichten (1,3) biostabil und/oder biodegradierbar und/oder mit Endothelzellen besiedelt sind.

5. Mehrschichtmaterial (4) gemäß einem der vorhergehenden Ansprüche,
**dadurch gekennzeichnet, dass** zumindest die Polymerbeschichtung einer äußeren Schicht und/oder eine äußere Schicht (1,3) eine Oberflächenmodifizierung und/oder eine Wirkstoffbeladung aufweist.

6. Mehrschichtmaterial (4) gemäß einem der vorhergehenden Ansprüche,
**dadurch gekennzeichnet, dass** die äußeren Schichten (1,3) in Form von Fasern und/oder Filmen und/oder Blends und/oder Folien und/oder Vliesen und/oder Kompositen ausgeführt sind.

7. Mehrschichtmaterial (4) gemäß einem der vorhergehenden Ansprüche,
**dadurch gekennzeichnet, dass** das flexibel ausgestaltete Metallgebilde ein- oder mehrlagig ist.

8. Mehrschichtmaterial (4) gemäß einem der vorhergehenden Ansprüche,
**dadurch gekennzeichnet, dass** das flexibel ausgestaltete Metallgebilde ein ultradünnes Gewebe und/oder Gewirk und/oder Geflecht und/oder Gelege und/oder Vlies und/oder Filz aus Drähten und/oder Fäden und/oder Filamenten und/oder Fasern und/oder Bändern und/oder Strängen und/oder Fibrillen, Hohlfasern, Verdrillungen, Komposite, Röhrchen, Nanoröhrchen, Koaxialfasern, -fäden, -filamente und/oder ummantelte Fasern ist.

9. Mehrschichtmaterial (4) gemäß Anspruch 9, **dadurch gekennzeichnet, dass** die Drähte und/oder Fäden und/oder Filamente und/oder Fasern und/oder Bänder und/oder Stränge und/oder Fibrillen eine Stärke im Mikrometerbereich aufweisen.

10. Verfahren zur Herstellung des Mehrschichtmaterials (4) gemäß Anspruch 1, aufweisend folgende Verfahrensschritte:
a. Herstellung des Metallgebildes,
b. Reinigung des Metallgebildes,
c. Beschichtung des Metallgebildes.

11. Anwendung des Mehrschichtmaterials (4) gemäß Anspruch 1 als Filter, Ventilbauteil oder temporäre und/oder permanente Implantatanwendung bei Knochendefekten.

12. Medizinisches kardiovaskuläres Implantat aufweisend ein Mehrschichtmaterial (4) gemäß Anspruch 1, wobei das Mehrschichtmaterial (4) eine Segelform aufweist und wobei das Mehrschichtmaterial (4) zumindest eine vordefinierte fixierte Falte (7) aufweist, um eine mechanische und strömungsdynamische Stabilität des medizinischen Implantates zu erreichen.

13. Medizinisches kardiovaskuläres Implantat gemäß Anspruch 12, **dadurch gekennzeichnet, dass** die zumindest eine vordefinierte fixierte Falte (7) asymmetrisch gebogen ist, um Totbereiche stagnierender Strömung zu vermeiden.

14. Medizinisches kardiovaskuläres Implantat gemäß Anspruch 12 oder 13,
**dadurch gekennzeichnet, dass** die Fixierung der zumindest einen vordefinierten Falte (7) formschlüssig und/oder kraftschlüssig und/oder stoffschlüssig ist.

15. Medizinisches kardiovaskuläres Implantat gemäß einem der Ansprüche 12 bis 14, **dadurch gekennzeichnet, dass** das medizinische kardiovaskuläre Implantat ein Okkluder oder eine Herzklappenprothese ist.

16. Medizinisches kardiovaskuläres Implantat gemäß einem der Ansprüche 12 bis 15, **dadurch gekennzeichnet, dass** das medizinische kardiovaskuläre Implantat eine Einsegelklappe oder Mehrsegelklappe ist.

17. Medizinisches kardiovaskuläres Implantat gemäß Anspruch 16, **dadurch gekennzeichnet, dass** die Einsegelklappe oder Mehrsegelklappe zur offenchirurgischen und Transkatheter-Aortenklappenimplantation vorgesehen ist.

18. Verfahren zur Herstellung des medizinischen kardiovaskulären Implantats gemäß Anspruch 12, aufweisend folgende Verfahrensschritte:
a. Herstellung eines Mehrschichtmaterials (4) nach Anspruch 1 durch
i. Herstellung des Metallgebildes,
ii. Reinigung des Metallgebildes,
iii. Beschichtung des Metallgebildes,
b. Zuschnitt des Mehrschichtmaterials (4),
c. Erzeugen zumindest einer vordefinierten fixierten Falte (7) im Mehrschichtmaterial (4) durch Legung der Falte, Fixierung der Falte und Erzeugung einer Vorbiegung der Falte.

19. Verfahren zur Herstellung eines medizinischen kardiovaskulären Implantats gemäß Anspruch 18, **dadurch gekennzeichnet, dass** die Erzeugung der zumindest einen vordefinierten Falte (7) erfolgt, indem das Mehrschichtmaterial (4) geknickt, fixiert und nach einer Seite vorgebogen wird.
